# EUROPÄISCHE PATENTSCHRIFT

(11) **EP 0 632 263 B1**
(45) Veröffentlichungstag und Bekanntmachung des Hinweises auf die Patenterteilung: **15.04.1998**
(21) Anmeldenummer: 94108154.9
(22) Anmeldetag: 26.05.1994
(51) Int. Cl.: G01N 21/53, G01M 15/00

(54) **Verfahren zur Untersuchung eines Gases**
Method for analyzing a gas
Procédé pour examiner un gas

(30) Priorität: 30.06.1993 DE 4321717
(43) Veröffentlichungstag der Anmeldung: 04.01.1995
(73) Patentinhaber: ROBERT BOSCH GMBH, 70442 Stuttgart (DE)
(72) Erfinder: Adolph, Dietrich, Dr. Dipl.-Phys., D-73095 Albershausen (DE)

(56) Entgegenhaltungen:
- DE-A- 3 204 456
- PATENT ABSTRACTS OF JAPAN vol. 10, no. 89 (P-444) 8. April 1986 & JP-A-60 225 029 (MAZDA KK) 9. November 1985
- PATENT ABSTRACTS OF JAPAN vol. 11, no. 173 (P-582) 4. Juni 1987 & JP-A-62 005 135 (MAZDA MOTOR CORP) 12. Januar 1987

## Beschreibung

Die Erfindung geht aus von einem Verfahren zur Untersuchung eines Gases nach der Gattung des Hauptanspruchs. In den USA wird ein Meßverfahren eingesetzt, das die Abgaskonzentrationen von vorbeifahrenden, von Otto-Motoren angetriebenen Kraftfahrzeugen ermittelt. Eine quer zur Fahrbahn aufgebaute optische Meßstrecke ermittelt die CO-Konzentration des Abgases. Bei diesem Meßverfahren sind weder die Absorptionslänge, die der Ausdehnung der Abgaswolke entspricht, noch die momentane Verdünnung der Abgaswolke durch die Umgebungsluft bekannt. Die gemessene CO-Konzentration wird mit einem Wert korrigiert, der aus einer CO₂-Konzentrationsmessung erhalten wird. Die CO₂-Messung wird ebenfalls in der optischen Meßstrecke vorgenommen. Ausgenützt wird die Tatsache, daß die CO₂-Konzentration im Abgas von Otto-Motoren stets wenigstens näherungsweise bei 15 % liegt. Unter der in der Praxis erfüllten Voraussetzung, daß sich die beiden Gaskomponenten CO und CO₂ gleichzeitig und gleichmäßig verdünnen, liefert das bekannte Verfahren ein Ergebnis für die CO-Konzentration des Abgases von Otto-Motoren, das eine ausreichende Genauigkeit aufweist.

Aus einer Firmenschrift der Robert Bosch GmbH, "Bosch-Diesel-Rauchgas-Meßgerät RTT 100/110 (Trübungsmeßgerät)", IA4/VPT-WaW 4470 D-8.92-15g, vom August 1992 ist ein gattungsgemäßes Verfahren bekannt, das zur Bestimmung des Rußgehaltes in Abgasen von Diesel-Brennkraftmaschinen eingesetzt wird. Das zu untersuchende Gas ist in einer Meßanordnung eine vorgegebene Strecke im wesentlichen parallel zu einer optischen Strahlung geführt. Ausgewertet wird der Einfluß des Gases auf die optische Strahlung, die eine Reduzierung der Intensität erfährt, die durch den im Abgas enthaltene Ruß verursacht ist. Der im Abgas enthaltene Ruß, dessen Konzentration zu ermitteln ist, kann eine Verschmutzung der im Meßgerät enthaltenen Meßkammer sowie insbesondere der im Meßgerät enthaltenen optischen Komponenten verursachen. Eine Verschmutzung kann auch beispielsweise durch abgeriebene Metallpartikel, Kraftstoffadditive, Schwefelverbindungen oder beispielsweise durch in Wassertröpfchen gelöste Stoffe auftreten. Bei dem bekannten Meßgerät sind Heizungen vorgesehen, die jeweils an der Übergangsstelle von der Meßkammer zu den optischen Komponenten angeordnet sind. Die Temperatur der Heizungen ist so hoch vorzugeben, daß ein Abbrand der Verschmutzungen auftritt. Schwierigkeiten bereiten diejenigen Bestandteile der Verschmutzung, die bei realisierbaren Temperaturen nicht ohne weiteres abbrennbar sind. Eine mechanische Reinigung nach einer vorgegebenen Betriebszeit der Meßkammer kann daher erforderlich sein.

Andere bekannte Meßgeräte zur Untersuchung eines Gases sehen den Einsatz von Spülluft vor, um einer Verschmutzung über einen längeren Zeitraum entgegenzuwirken. Eine Vermischung von Spülluft mit dem zu untersuchenden Gas bringt aber stets den Nachteil mit sich, daß die Meßunsicherheit erhöht ist. Der ungünstige Einfluß auf das Meßergebnis tritt insbesondere auf, wenn der Druck des zu untersuchenden Gases Änderungen, insbesondere impulsförmigen Änderungen unterworfen ist. Die Meßstrecke ist in diesen Betriebszuständen nicht mehr eindeutig definiert.

Der Erfindung liegt die Aufgabe zugrunde, ein Verfahren zur Untersuchung eines Gases anzugeben, bei dem die Verschmutzung einer Meßanordnung, durch die das zu untersuchende Gas geleitet wird, reduziert ist.

Die Aufgabe wird durch die im unabhängigen Anspruch angegebenen Merkmale gelöst.

### Vorteile der Erfindung

Das erfindungsgemäße Verfahren sieht vor, daß das Gas in verdünnter Form untersucht wird. Die Verdünnung reduziert die Verschmutzung der optischen Komponenten. Die Verdünnung hat auch eine geringere thermische Belastung der Meßanordnung zur Folge, wenn das zu untersuchende Gas, beispielsweise ein Abgas einer Diesel-Brennkraftmaschine, eine erhöhte Temperatur aufweist. Der Grad der Verdünnung des zu untersuchenden Gases wird dadurch berücksichtigt, daß durch zusätzliche Messungen die CO₂-Konzentrationen vor und nach dem Beimischen von Umgebungsluft ermittelt werden. Die zusätzliche Messung von CO₂ ist preiswert unter Zugrundelegung einer Infrarotabsorptionsmessung durchführbar, da CO₂ ein kräftiges und einfaches Infrarotabsorptionsspektrum aufweist. Das von der Meßanordnung ermittelte Untersuchungsergebnis wird anschließend in Abhängigkeit von den beiden Ergebnissen der CO₂-Konzentrationsmessungen korrigiert. Der Korrekturfaktor wird erhalten aus einer Verhältnisbildung der beiden CO₂-Konzentrationsmessungen.

Das erfindungsgemäße Verfahren ist geeignet zur Untersuchung von partikelhaltigen Gasen, die zu einer Verschmutzung der Meßanordnung führen könnten. Das erfindungsgemäße Verfahren ist insbesondere geeignet zur Untersuchung von Abgasen einer Dieselbrennkraftmaschine.

Vorteilhafte Weiterbildungen und Verbesserungen des erfindungsgemäßen Verfahrens ergeben sich aus Unteransprüchen.

Der Bereich der Beimischung von Umgebungsluft zum unverdünnten Gas liegt zwischen 1:1 bis 1:450. Unter Berücksichtigung der Tatsache, daß der CO₂-Anteil in der Abgaskonzentration einer Diesel-Brennkraftmaschine je nach Belastung des Motors zwischen ungefähr 3,5 Vol.% und 12,0 Vol.% liegt, ergibt sich eine Untergrenze der Beimischung von etwa 15 Vol.% im unverdünnten Abgas. Eine Obergrenze ist durch die CO₂-Konzentration der Umgebungsluft gegeben, die bei 330 ppm liegt. Bedingt durch die Empfindlichkeit einer bekannten CO₂-Meßstrecke im Vergleich zur CO₂-Konzentration der Umgebungsluft ergibt eine praktische Grenze des Bereichs von etwa 1:1 bis 1:100.

Weitere vorteilhafte Weiterbildungen sowie Ausgestaltungen des erfindungsgemäßen Verfahrens ergeben sich aus weiteren Unteransprüchen in Verbindung mit der folgenden Beschreibung.

### Zeichnung

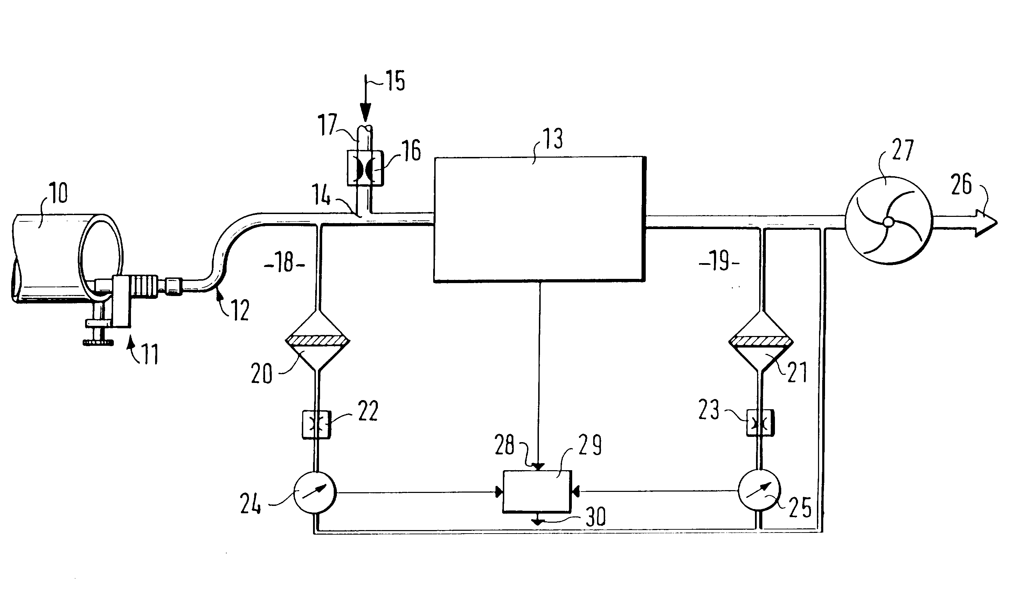

Die Figur zeigt eine Vorrichtung zur Untersuchung eines Gases, die zur Realisierung des erfindungsgemäßen Verfahrens geeignet ist.

In der Figur ist ein Endstück eines Auspuffrohres 10 einer nicht näher gezeigten Brennkraftmaschine gezeigt, an dem eine Sonde 11 befestigt ist, die einen Teil des Abgasstroms als zu untersuchendes Gas aufnimmt. Das zu untersuchende Gas ist in einem Schlauch 12 zu einer Meßanordnung 13 geführt. Im Schlauch 12 ist eine Beimischung 14 von Umgebungsluft 15 zum zu untersuchenden Gas vorgesehen. Das Beimischungsverhältnis kann in einem weiten Bereich mit einer ersten Drossel 16 festgelegt werden, die in einem Zuführungskanal 17 für die Umgebungsluft 15 angeordnet ist.

Vor der Beimischung 14 ist ein erster Meßpfad 18 für eine erste CO₂-Konzentrationsmessung und nach der Beimischung 14 ein zweiter Meßpfad 19 für eine zweite CO₂-Konzentrationsmessung vorgesehen. Die beiden Meßpfade 18, 19 enthalten jeweils ein Partikelfilter 20, 21, jeweils eine Drossel 22, 23 sowie jeweils eine CO₂-Konzentrationsmeßvorrichtung 24, 25. Die beiden Meßpfade 18, 19 führen zu einem Auslaß 26, zu dem auch das zu untersuchende Gas nach der Meßanordnung 13 geführt ist. Vor dem Auslaß ist eine Pumpe oder ein Gebläse 27 angeordnet, das für einen kontinuierlichen Prüfgasdurchsatz sorgt.

Die Meßanordnung 13 gibt ein Untersuchungsergebnis 28 an eine signalverarbeitende Anordnung 29 ab, die aus dem Untersuchungsergebnis 28 und aus den von den CO₂-Konzentrationsmeßvorrichtungen 24, 25 abgegebenen Signalen ein korrigiertes Meßergebnis 30 ermittelt.

Das erfindungsgemäße Verfahren wird anhand der in der Figur gezeigten Vorrichtung näher erläutert:

Die Sonde 11 erfaßt ein zu untersuchendes Gas, das der Meßanordnung 13 zugeführt wird. Im gezeigten Ausführungsbeispiel ist das zu untersuchende Gas das Abgas einer Diesel-Brennkraftmaschine, das über das Auspuffrohr 10 ins Freie geführt ist. Das Abgas einer Diesel-Brennkraftmaschine enthält beispielsweise Metallpartikel, Kraftstoffadditive, Schwefelverbindungen oder auch in Wassertröpfchen gelöste Stoffe sowie insbesondere Ruß. Die Meßanordnung 13 ist beispielsweise ein Meßgerät zum Ermitteln des Rußgehaltes im Abgas einer Diesel-Brennkraftmaschine. Insbesondere durch die im zu untersuchenden Gas enthaltenen Partikel besteht prinzipiell ein Verschmutzungsproblem für die Meßanordnung 13.

Die Meßanordnung 13 ist beispielsweise ein Trübungsmeßgerät, das eingangs im Stand der Technik beschrieben wurde. Anstelle eines Trübungsmeßgeräts ist beispielsweise auch ein Streulichtmeßgerät oder eine andere geeignete Vorrichtung einsetzbar.

Einer Reduzierung der Verschmutzung durch Partikel wird mit einer Verdünnung des zu untersuchenden Gases entgegengewirkt. Das erfindungsgemäße Verfahren sieht deshalb zunächst die Beimischung von Umgebungsluft 15 zum zu untersuchenden Gas vor. Prinzipiell könnte die Verdünnung durch entsprechende Vorrichtungen zur Messung des Volumendurchflusses ermittelt werden. Erfindungsgemäß ist aber vorgesehen, in der signalverarbeitenden Anordnung 29 aus den von den beiden CO₂-Konzentrationsmeßvorrichtungen 24, 25 abgegebenen Werten und aus dem von der Meßanordnung 13 ermittelten Untersuchungsergebnis 28 das korrigierte Meßergebnis 30 zu ermitteln. Wesentlich für die Durchführung des erfindungsgemäßen Verfahrens ist die Messung der CO₂-Konzentration sowohl vor der Beimischung 14 als auch nach der Beimischung 14. Der Korrekturfaktor ergibt sich aus dem Verhältnis der von den CO₂-Konzentrationsmeßvorrichtungen 24, 25 abgegebenen Werte.

Im gezeigten Ausführungsbeispiel ist der zweite Meßpfad 19 nach der Meßanordnung 13 gezeigt. In einer gleichwertigen Ausführung ist es auch möglich, den zweiten Meßpfad 19 nach der Beimischung 14 aber noch vor der Meßanordnung 13 anzuordnen.

Die in den beiden Meßpfaden 18, 19 angeordneten Partikelfilter 20, 21 halten die gegebenenfalls vorhandenen Partikel von den CO₂-Konzentrationsmeßvorrichtungen 24, 25 fern. Der Durchfluß in den beiden Meßpfaden 18, 19 kann mit den Drosseln 22, 23 auf ein vorgegebenes Maß festgelegt werden. Die beiden CO₂-Konzentrationsmeßvorrichtungen 24, 25 arbeiten vorzugsweise mit einer Infrarotabsorption, da CO₂ ein ausgeprägtes und einfaches InfrarotabsorptionsSPektrUm aufweist. Die CO₂-Konzentrationsmessung ist deshalb mit einfachen Mitteln bei hoher Genauigkeit durchführbar.

Der zweite Meßpfad 19 zur Messung der CO₂-Konzentration im verdünnten zu untersuchenden Gas wäre allein im allgemeinen nicht ausreichend, weil die CO₂-Konzentration im zu untersuchenden Gas Änderungen unterworfen sein kann. Ein korrektes Meßergebnis 30 wird deshalb nur durch die Messung der CO₂-Konzentration auch im unverdünnten zu untersuchenden Gas möglich.

Der Anteil der Beimischung 14 der Umgebungsluft 15 zum zu untersuchenden Gas hängt ab von der Meßanordnung 13, insbesondere von der Leistung des Gebläses 27, und von der Zusammensetzung des zu untersuchenden Gases. Vorgegeben ist die Konzentration von CO₂ in der Umgebungsluft, die bei 330 ppm liegt. Die CO₂-Konzentration im Abgas von Diesel-Brennkraftmaschinen liegt im Bereich von ungefähr 3,5 Vol.% bis ungefähr 12,0 Vol.%. Für die Untersuchung beispielsweise des Abgases einer Diesel-Brennkraftmaschine ist daher die Beimischung 14 von Umgebungsluft 15 zum zu untersuchenden Gas in einem Bereich von 1:1 bis 1:450 möglich. Die Untergrenze der Verdünnung ist etwa 15 Vol.% im unverdünnten Abgas, während die Obergrenze bei 330 ppm für CO₂ liegt, die dem CO₂-Gehalt der Umgebungsluft entspricht. In der Praxis wird der Bereich vorzugsweise festgelegt von 1:1 bis 1:100, der durch die Eigenschaften sowohl der Meßanordnung 13 als auch die der CO₂-Konzentrationsmeßvorrichtungen 24, 25 gegeben ist.

Die angegebenen Zahlenwerte, insbesondere auch die Untergrenze der Verdünnung von ungefähr 15 Vol.% im unverdünnten Abgas sind auch geeignet bei Meßanordnungen 13, die das Abgas einer Ölheizungsanlage untersuchen. Bei diesem Verbrennungsvorgang liegt die maximale CO₂-Konzentration im Abgas bei näherungsweise stöchiometrischer Verbrennung bei ungefähr 15 Vol.%

## Patentansprüche

1. Verfahren zur Untersuchung eines Gases, mit einer Meßanordnung, durch die das zu untersuchende Gas geleitet wird, dadurch gekennzeichnet, daß zunächst der CO₂-Gehalt des zu untersuchenden Gases ermittelt wird, daß danach dem zu untersuchenden Gas Umgebungsluft (15) beigemischt wird, daß anschließend wieder der CO₂-Gehalt des verdünnten, zu untersuchenden Gases ermittelt wird und daß das von einer Meßanordnung (13) ermittelte Untersuchungsergebnis (28) in einer signalverarbeitenden Anordnung (29) in Abhängigkeit von den beiden Ergebnissen der CO₂-Ermittlungen korrigiert wird.

2. Verfahren nach Anspruch 1, dadurch gekennzeichnet, daß das Verhältnis aus den beiden Ergebnissen der CO₂-Ermittlungen berechnet wird.

3. Verfahren nach Anspruch 1, dadurch gekennzeichnet, daß die Beimischung im Bereich von 1:1 bis 1:450, vorzugsweise bis 1:100 liegt.

4. Verfahren nach Anspruch 1, dadurch gekennzeichnet, daß die Meßanordnung (13) eine optische Strecke enthält, die das zu untersuchende Gas durchläuft.

5. Verfahren nach Anspruch 4, dadurch gekennzeichnet, daß die Meßanordnung (13) eine Trübungsmessung und/oder eine Streulichtmessung vornimmt.

6. Verfahren nach Anspruch 1, gekennzeichnet durch die Verwendung zur Untersuchung von rußhaltigen Abgasen.

7. Verfahren nach Anspruch 6, dadurch gekennzeichnet, daß als zu untersuchendes Gas das Abgas einer Diesel-Brennkraftmaschine vorgesehen ist.

## Claims

1. Method for analyzing a gas, having a measuring arrangement through which the gas to be analyzed is conducted, characterized in that firstly the CO₂ content of the gas to be analyzed is determined, in that ambient air (15) is then mixed in with the gas to be analyzed, in that subsequently the CO₂ content of the diluted gas to be analyzed is determined again, and in that the result (28) of the analysis determined by a measuring arrangement (13) is corrected in a signal-processing arrangement (29) as a function of the two results of the CO₂ determinations.

2. Method according to Claim 1, characterized in that the ratio of the two results of the CO₂ determinations is calculated.

3. Method according to Claim 1, characterized in that the mixture is within the range of 1:1 to 1:450, preferably up to 1:100.

4. Method according to Claim 1, characterized in that the measuring arrangement (13) contains an optical route through which the gas to be analyzed runs.

5. Method according to Claim 4, characterized in that the measuring arrangement (13) carries out a turbidity measurement and/or a scattered-light measurement.

6. Method according to Claim 1, characterized by the use for analyzing exhaust gases containing soot.

7. Method according to Claim 6, characterized in that the exhaust gas of a diesel internal combustion engine is provided as gas to be analyzed.

## Revendications

1. Procédé d'analyse d'un gaz à l'aide d'une installation de mesure traversée par le gaz à analyser,
caractérisé en ce qu'
• on détermine tout d'abord la teneur en CO₂ du gaz d'analyse,
• on mélange de l'air ambiant (15) au gaz d'analyse,
• on détermine de nouveau la teneur en CO₂ du gaz à analyser dilué et,
• on corrige le résultat d'analyse (28) fourni par un dispositif de mesure (13) dans un dispositif de traitement de signal (29) en fonction des deux résultats de détermination de la teneur en CO₂.

2. Procédé selon la revendication 1,
caractérisé en ce qu'
on calcule le rapport des deux résultats des déterminations de CO₂.

3. Procédé selon la revendication 1,
caractérisé en ce que
le rapport de mélange est compris entre 1:1 et 1:450 et situé de préférence à 1:100.

4. Procédé selon la revendication 1,
caractérisé en ce que
le dispositif de mesure (13) comporte un chemin optique traversé par le gaz d'analyse.

5. Procédé selon la revendication 4,
caractérisé en ce que
le dispositif de mesure (13) effectue une mesure de turbidité et/ou une mesure de lumière dispersée.

6. Procédé selon la revendication 1,
caractérisé en ce qu'
on utilise l'analyse de gaz d'échappement contenant du noir de fumée.

7. Procédé selon la revendication 6,
caractérisé en ce que
le gaz à analyser est le gaz d'échappement d'un moteur à combustion interne Diesel.
